# EUROPÄISCHE PATENTSCHRIFT

(11) **EP 1 106 684 B1**
(45) Veröffentlichungstag und Bekanntmachung des Hinweises auf die Patenterteilung: **17.05.2006**
(21) Anmeldenummer: 00125831.8
(22) Anmeldetag: 25.11.2000
(51) Int. Cl.: C12N 9/02, C12P 13/08

(54) **Polynukleotidsequenzen aus Corynebacterium glutamicum, die Succinatdehydrogenase-Untereinheiten (sdhA, sdhB, sdhC) kodierenden**
Polynucleotide sequences from Corynebacterium glutamicum coding for succinate dehydrogenase subunits (sdhA, sdhB, sdhC)
Séquences polynucleotidiques de Corynebacterium glutamicum codant pour des sous-unités de succinate déshydrogénase

(30) Priorität: 10.12.1999 DE 19959650
(43) Veröffentlichungstag der Anmeldung: 13.06.2001
(73) Patentinhaber: Degussa AG, 40474 Düsseldorf (DE)
(72) Erfinder: Möckel, Bettina, Dr., 40597 Düsseldorf (DE); Pfefferle, Walter, Dr., 33790 Halle (West.) (DE); Marx, Achim, Dr., 33613 Bielefeld (DE)

(56) Entgegenhaltungen:
- WO-A-01/00844
- WO-A-01/02544
- WO-A-99/27105
- ITO M. ET AL.: "SUCCINATE DEHYDROGENASE CYTOCHROME B-558 SUBUNIT (FRAGMENT)" EMBL DATABASE ENTRY O54447; ACCESSION NO. O54447,1. Juni 1998 (1998-06-01), XP002163653
- KALMAN S ET AL: "COMPARATIVE GENOMES OF CHLAMYDIA PNEUMONIAE AND C. TRACHOMATIS" NATURE GENETICS, Bd. 21, Nr. 4, April 1999 (1999-04), Seiten 385-389, XP000853883 ISSN: 1061-4036

### SEQUENZPROTOKOLL

<110> Degussa-Hüls AG
<120> Neue für die Gene sdhA, sdhB und sdhC codierende Nucleotidsequenzen
<130> 990170 BT
<140>
   <141>
<160> 7
<170> PatentIn Ver. 2.1
<210> 1
   <211> 4080
   <212> DNA
   <213> Corynebacterium glutamicum
<220>
   <221> gene
   <222> (288)..(1169)
   <223> sdhC
<220>
   <221> gene
   <222> (1330)..(3207)
   <223> sdhA
<220>
   <221> gene
   <222> (3102)..(3956)
   <223> sdhB
<400> 1
   gtgcccggcg tggtcgggcc acatccgccc cgggaacttt ttaggcacct acggtgcaac 60
   tgttgggata attgtgtcac ctgcgcaaag ttgctccctg gatcggaagg ttgggctgtc 120
   taaacttttt ggttgatacc aaacggggtt agaaactgtt cggatcggta tcctgtgagg 180
   aagctcacct tggttttaga atgttgaaaa ggcctcacgt ttccgcaggt agagcacact 240
   caattaaatg agcgtcaaac gacaataaag taaggctatc ctaataagtg gggttttatg 300
   tctctaaaca gccagttggg ggtcatgggg gagcgccccg tgactggtta atgccccgat 360
   ctgggacgta cagtaacaac gacactggag gtgccatgac tgttagaaat cccgaccgtg 420
   aggcaatccg tcacggaaaa attacgacgg aggcgctgcg tgagcgtccc gcatacccga 480
   cctgggcaat gaagctgacc atggccatca ctggcctaat gtttggtggc ttcgttcttg 540
   ttcacatgat cggaaacctg aaaatcttca tgccggacta cgcagccgat tctgcgcatc 600
   cgggtgaagc acaagtagat gtctacggcg agttcctgcg tgagatcgga tccccgatcc 660
   tcccacacgg ctcagtcctc tggatcctac gtattatcct gctggtcgca ttggttctgc 720
   acatctactg tgcattcgca ttgaccggcc gttctcacca gtcccgcgga aagttccgcc 780
   gtaccaacct cgttggcggc ttcaactcct tcgcgacccg ctccatgctg gtgaccggaa 840
   tcgttctcct tgcgttcatt atcttccaca tcctcgacct gaccatgggt gttgctccag 900
   cagccccaac ctcattcgag cacggcgaag tatacgcaaa catggtggct tcctttagcc 960
   gctggcctgt agcaatttgg tacatcattg ccaacctggt cctgttcgtc cacctgtcac 1020
   acggcatctg gcttgcagtc tctgacctgg gaatcaccgg acgccgctgg agggcaatcc 1080
   tcctcgcagt tgcgtacatc gttcctgcac tggtcctgat cggcaacatc accattccgt 1140
   tcgccatcgc tgttggctgg attgcgtaaa ggttaggaag aatttatgag cactcactct 1200
   gaaaccaccc gcccagagtt catccaccca gtctcagtcc tcccagaggt ctcagctggt 1260
   acggtccttg acgctgcaga gccagcaggc gttcccacca aagatatgtg ggaataccaa 1320
   aaagaccaca tgaacctggt ctccccactg aaccgacgca agttccgtgt cctcgtcgtt 1380
   ggcaccggcc tgtccggtgg tgctgcagca gcagccctcg gcgaactcgg atacgacgtc 1440
   aaggcgttca cctaccacga cgcacctcgc cgtgcgcact ccattgctgc acagggtggc 1500
   gttaactccg cccgcggcaa gaaggtagac aacgacggcg cataccgcca cgtcaaggac 1560
   accgtcaagg gcggcgacta ccgtggtcgc gagtccgact gctggcgtct cgccgtcgag 1620
   tccgtccgcg tcatcgacca catgaacgcc atcggtgcac cattcgcccg cgaatacggt 1680
   ggcgccttgg caacccgttc cttcggtggt gtgcaggtct cccgtaccta ctacacccgt 1740
   ggacaaaccg gacagcagct gcagctctcc accgcatccg cactacagcg ccagatccac 1800
   ctcggctccg tagaaatctt cacccataac gaaatggttg acgtcattgt caccgaacgt 1860
   aacggtgaaa agcgctgcga aggcctgatc atgcgcaacc tgatcaccgg cgagctcacc 1920
   gcacacaccg gccatgccgt tatcctggca accggtggct acggcaacgt gtaccacatg 1980
   tccaccctgg ccaagaactc caacgcctcg gccatcatgc gtgcatacga agccggcgca 2040
   tacttcgcgt ccccatcgtt catccagttc cacccaaccg gcctgcctgt gaactccacc 2100
   tggcagtcca agaccattct gatgtccgag tcgctgcgta acgacggccg catctggtcc 2160
   cctaaggaac cgaacgataa ccgcgatcca aacaccatcc ctgaggatga gcgcgactac 2220
   ttcctggagc gccgctaccc agcattcggt aacctcgtcc cacgtgacgt tgcttcccgt 2280
   gcgatctccc agcagatcaa tgctggtctc ggtgttggac ctctgaacaa cgctgcatac 2340
   ctggacttcc gcgacgccac cgagcgcctc ggacaggaca ccatccgcga gcgttactcc 2400
   aacctcttca ccatgtacga agaggcaatt ggcgaggacc catactccag cccaatgcgt 2460
   attgcaccga cctgccactt caccatgggt ggcctctgga ctgacttcaa cgaaatgacg 2520
   tcactcccag gtctgttctg cgcaggcgaa gcatcctgga cctaccacgg tgcaaaccgt 2580
   ctgggcgcaa actccctgct ctccgcttcc gtcgatggct ggttcaccct gccattcacc 2640
   atccctaact acctcggccc attgcttggc tccgagcgtc tgtcagagga tgcaccagaa 2700
   gcacaggcag cgattgcgcg tgcacaggct cgcattgacc gcctcatggg caaccgccca 2760
   gagtgggtcg gtgacaacgt tcacggacct gagtactacc accgccagct tggcgatatc 2820
   ctgtacttct cctgtggcgt ttcccgaaac gtagaagacc tccaggatgg catcaacaag 2880
   atccgtgccc tccgcgatga cttctggaag aacatgcgca tcaccggcag caccgatgag 2940
   atgaaccagg ttctcgaata cgcagcacgc gtagccgact acatcgacct cggcgaactc 3000
   atgtgtgtcg acgccctcga ccgcgacgag tcctgtggcg ctcacttccg cgacgaccac 3060
   ctctccgaag atggcgaagc agaacgtgac gacgaaaact ggtgcttcgt ctccgcatgg 3120
   gaaccaggcg agaacggaac cttcgtccgc cacgcagaac cactgttctt cgaatccgtc 3180
   ccactgcaga caaggaacta caagtaatga aacttacact tgagatctgg cgtcaagcag 3240
   gcccaactgc ggaaggcaag ttcgaaaccg tccaggttga cgacgccgtc gcgcagatgt 3300
   ccatcctgga gctgcttgac cacgtaaaca acaagttcat cgaagaaggc aaagaaccat 3360
   tcgcgttcgc ctctgactgc cgcgaaggca tttgtggtac ctgtggtctc ctcgtgaacg 3420
   gtcgccctca cggcgccgac cagaacaagc ctgcctgtgc gcagcgcctg gtcagctaca 3480
   aggaaggcga caccctcaag atcgaaccac tgcgttccgc cgcataccca gtgatcaagg 3540
   acatggtcgt cgaccgctcc gcactggacc gtgtcatgga acagggtggc tacgtgacca 3600
   tcaacgcagg taccgcacct gacgctgata ccctccacgt caaccacgaa accgcagaac 3660
   tcgcacttga ccacgcagcc tgcatcggct gtggcgcatg tgttgctgcc tgccctaacg 3720
   gcgcagcaca cctgttcacc ggcgcaaagc ttgttcacct ctccctcctc ccactgggta 3780
   aggaagagcg cggactgcgt gcacgtaaga tggttgatga aatggaaacc aacttcggac 3840
   actgctccct ctacggcgag tgcgcagatg tctgccccgc aggcatccca ctgaccgctg 3900
   tggcagctgt caccaaggaa cgtgcgcgtg cagctttccg aggcaaagac gactagtctt 3960
   taatccaagt aagtaccggt tcagacagtt aaaccagaaa gacgagtgaa caccatgtcc 4020
   tccgcgaaaa agaaacccgc accggagcgt atgcactaca tcaagggcta tgtacctgtg 4080
<210> 2
   <211> 882
   <212> DNA
   <213> Corynebacterium glutamicum
<220>
   <221> CDS
   <222> (1)..(879)
   <223> sdhC
<400> 2
   gtg ggg ttt tat gtc tct aaa cag cca gtt ggg ggt cat ggg gga gcg     48
   Val Gly Phe Tyr Val Ser Lys Gln Pro Val Gly Gly His Gly Gly Ala
    1               5                  10                  15
   ccc cgt gac tgg tta atg ccc cga tct ggg acg tac agt aac aac gac     96
   Pro Arg Asp Trp Leu Met Pro Arg Ser Gly Thr Tyr Ser Asn Asn Asp
               20                  25                  30
   act gga ggt gcc atg act gtt aga aat ccc gac cgt gag gca atc cgt    144
   Thr Gly Gly Ala Met Thr Val Arg Asn Pro Asp Arg Glu Ala Ile Arg
           35                  40                  45
   cac gga aaa att acg acg gag gcg ctg cgt gag cgt ccc gca tac ccg    192
   His Gly Lys Ile Thr Thr Glu Ala Leu Arg Glu Arg Pro Ala Tyr Pro
       50                  55                  60
   acc tgg gca atg aag ctg acc atg gcc atc act ggc cta atg ttt ggt    240
   Thr Trp Ala Met Lys Leu Thr Met Ala Ile Thr Gly Leu Met Phe Gly
   65                  70                  75                  80
   ggc ttc gtt ctt gtt cac atg atc gga aac ctg aaa atc ttc atg ccg    288
   Gly Phe Val Leu Val His Met Ile Gly Asn Leu Lys Ile Phe Met Pro
                   85                  90                  95
   gac tac gca gcc gat tct gcg cat ccg ggt gaa gca caa gta gat gtc    336
   Asp Tyr Ala Ala Asp Ser Ala His Pro Gly Glu Ala Gln Val Asp Val
               100                 105                 110
   tac ggc gag ttc ctg cgt gag atc gga tcc ccg atc ctc cca cac ggc    384
   Tyr Gly Glu Phe Leu Arg Glu Ile Gly Ser Pro Ile Leu Pro His Gly
           115                 120                 125
   tca gtc ctc tgg atc cta cgt att atc ctg ctg gtc gca ttg gtt ctg    432
   Ser Val Leu Trp Ile Leu Arg Ile Ile Leu Leu Val Ala Leu Val Leu
       130                 135                 140
   cac atc tac tgt gca ttc gca ttg acc ggc cgt tct cac cag tcc cgc    480
   His Ile Tyr Cys Ala Phe Ala Leu Thr Gly Arg Ser His Gln Ser Arg
   145                 150                 155                 160
   gga aag ttc cgc cgt acc aac ctc gtt ggc ggc ttc aac tcc ttc gcg    528
   Gly Lys Phe Arg Arg Thr Asn Leu Val Gly Gly Phe Asn Ser Phe Ala
                   165                 170                 175
   acc cgc tcc atg ctg gtg acc gga atc gtt ctc ctt gcg ttc att atc    576
   Thr Arg Ser Met Leu Val Thr Gly Ile Val Leu Leu Ala Phe Ile Ile
               180                 185                 190
   ttc cac atc ctc gac ctg acc atg ggt gtt gct cca gca gcc cca acc    624
   Phe His Ile Leu Asp Leu Thr Met Gly Val Ala Pro Ala Ala Pro Thr
           195                 200                 205
   tca ttc gag cac ggc gaa gta tac gca aac atg gtg gct tcc ttt agc    672
   Ser Phe Glu His Gly Glu Val Tyr Ala Asn Met Val Ala Ser Phe Ser
       210                 215                 220
   cgc tgg cct gta gca att tgg tac atc att gcc aac ctg gtc ctg ttc    720
   Arg Trp Pro Val Ala Ile Trp Tyr Ile Ile Ala Asn Leu Val Leu Phe
   225                 230                 235                 240
   gtc cac ctg tca cac ggc atc tgg ctt gca gtc tct gac ctg gga atc    768
   Val His Leu Ser His Gly Ile Trp Leu Ala Val Ser Asp Leu Gly Ile
                   245                 250                 255
   acc gga cgc cgc tgg agg gca atc ctc ctc gca gtt gcg tac atc gtt    816
   Thr Gly Arg Arg Trp Arg Ala Ile Leu Leu Ala Val Ala Tyr Ile Val
               260                 265                 270
   cct gca ctg gtc ctg atc ggc aac atc acc att ccg ttc gcc atc gct    864
   Pro Ala Leu Val Leu Ile Gly Asn Ile Thr Ile Pro Phe Ala Ile Ala
           275                 280                 285
   gtt ggc tgg att gcg taa                                            882
   Val Gly Trp Ile Ala
       290
<210> 3
   <211> 293
   <212> PRT
   <213> Corynebacterium glutamicum
<400> 3
   Val Gly Phe Tyr Val Ser Lys Gln Pro Val Gly Gly His Gly Gly Ala
   1               5                   10                  15
   Pro Arg Asp Trp Leu Met Pro Arg Ser Gly Thr Tyr Ser Asn Asn Asp
               20                  25                  30
   Thr Gly Gly Ala Met Thr Val Arg Asn Pro Asp Arg Glu Ala Ile Arg
           35                  40                  45
   His Gly Lys Ile Thr Thr Glu Ala Leu Arg Glu Arg Pro Ala Tyr Pro
       50                  55                  60
   Thr Trp Ala Met Lys Leu Thr Met Ala Ile Thr Gly Leu Met Phe Gly
   65                  70                  75                  80
   Gly Phe Val Leu Val His Met Ile Gly Asn Leu Lys Ile Phe Met Pro
                   85                  90                  95
   Asp Tyr Ala Ala Asp Ser Ala His Pro Gly Glu Ala Gln Val Asp Val
               100                 105                 110
   Tyr Gly Glu Phe Leu Arg Glu Ile Gly Ser Pro Ile Leu Pro His Gly
           115                 120                 125
   Ser Val Leu Trp Ile Leu Arg Ile Ile Leu Leu Val Ala Leu Val Leu
       130                 135                 140
   His Ile Tyr Cys Ala Phe Ala Leu Thr Gly Arg Ser His Gln Ser Arg
   145                 150                 155                 160
   Gly Lys Phe Arg Arg Thr Asn Leu Val Gly Gly Phe Asn Ser Phe Ala
                   165                 170                 175
   Thr Arg Ser Met Leu Val Thr Gly Ile Val Leu Leu Ala Phe Ile Ile
               180                 185                 190
   Phe His Ile Leu Asp Leu Thr Met Gly Val Ala Pro Ala Ala Pro Thr
           195                 200                 205
   Ser Phe Glu His Gly Glu Val Tyr Ala Asn Met Val Ala Ser Phe Ser
       210                 215                 220
   Arg Trp Pro Val Ala Ile Trp Tyr Ile Ile Ala Asn Leu Val Leu Phe
   225                 230                 235                 240
   Val His Leu Ser His Gly Ile Trp Leu Ala Val Ser Asp Leu Gly Ile
                   245                 250                 255
   Thr Gly Arg Arg Trp Arg Ala Ile Leu Leu Ala Val Ala Tyr Ile Val
               260                 265                 270
   Pro Ala Leu Val Leu Ile Gly Asn Ile Thr Ile Pro Phe Ala Ile Ala
           275                 280                 285
   Val Gly Trp Ile Ala
       290
<210> 4
   <211> 1878
   <212> DNA
   <213> Corynebacterium glutamicum
<220>
   <221> CDS
   <222> (1)..(1875)
   <223> sdhA
<400> 4
   atg aac ctg gtc tcc cca ctg aac cga cgc aag ttc cgt gtc ctc gtc      48
   Met Asn Leu Val Ser Pro Leu Asn Arg Arg Lys Phe Arg Val Leu Val
   1               5                   10                  15
   gtt ggc acc ggc ctg tcc ggt ggt gct gca gca gca gcc ctc ggc gaa      96
   Val Gly Thr Gly Leu Ser Gly Gly Ala Ala Ala Ala Ala Leu Gly Glu
               20                  25                  30
   ctc gga tac gac gtc aag gcg ttc acc tac cac gac gca cct cgc cgt     144
   Leu Gly Tyr Asp Val Lys Ala Phe Thr Tyr His Asp Ala Pro Arg Arg
           35                  40                  45
   gcg cac tcc att gct gca cag ggt ggc gtt aac tcc gcc cgc ggc aag     192
   Ala His Ser Ile Ala Ala Gln Gly Gly Val Asn Ser Ala Arg Gly Lys
       50                  55                  60
   aag gta gac aac gac ggc gca tac cgc cac gtc aag gac acc gtc aag     240
   Lys Val Asp Asn Asp Gly Ala Tyr Arg His Val Lys Asp Thr Val Lys
   65                  70                  75                  80
   ggc ggc gac tac cgt ggt cgc gag tcc gac tgc tgg cgt ctc gcc gtc     288
   Gly Gly Asp Tyr Arg Gly Arg Glu Ser Asp Cys Trp Arg Leu Ala Val
                   85                  90                  95
   gag tcc gtc cgc gtc atc gac cac atg aac gcc atc ggt gca cca ttc     336
   Glu Ser Val Arg Val Ile Asp His Met Asn Ala Ile Gly Ala Pro Phe
               100                 105                 110
   gcc cgc gaa tac ggt ggc gcc ttg gca acc cgt tcc ttc ggt ggt gtg     384
   Ala Arg Glu Tyr Gly Gly Ala Leu Ala Thr Arg Ser Phe Gly Gly Val
           115                 120                 125
   cag gtc tcc cgt acc tac tac acc cgt gga caa acc gga cag cag ctg     432
   Gln Val Ser Arg Thr Tyr Tyr Thr Arg Gly Gln Thr Gly Gln Gln Leu
       130                 135                 140
   cag ctc tcc acc gca tcc gca cta cag cgc cag atc cac ctc ggc tcc     480
   Gln Leu Ser Thr Ala Ser Ala Leu Gln Arg Gln Ile His Leu Gly Ser
   145                 150                 155                 160
   gta gaa atc ttc acc cat aac gaa atg gtt gac gtc att gtc acc gaa     528
   Val Glu Ile Phe Thr His Asn Glu Met Val Asp Val Ile Val Thr Glu
                   165                 170                 175
   cgt aac ggt gaa aag cgc tgc gaa ggc ctg atc atg cgc aac ctg atc     576
   Arg Asn Gly Glu Lys Arg Cys Glu Gly Leu Ile Met Arg Asn Leu Ile
               180                 185                 190
   acc ggc gag ctc acc gca cac acc ggc cat gcc gtt atc ctg gca acc     624
   Thr Gly Glu Leu Thr Ala His Thr Gly His Ala Val Ile Leu Ala Thr
           195                 200                 205
   ggt ggc tac ggc aac gtg tac cac atg tcc acc ctg gcc aag aac tcc     672
   Gly Gly Tyr Gly Asn Val Tyr His Met Ser Thr Leu Ala Lys Asn Ser
       210                 215                 220
   aac gcc tcg gcc atc atg cgt gca tac gaa gcc ggc gca tac ttc gcg     720
   Asn Ala Ser Ala Ile Met Arg Ala Tyr Glu Ala Gly Ala Tyr Phe Ala
   225                 230                 235                 240
   tcc cca tcg ttc atc cag ttc cac cca acc ggc ctg cct gtg aac tcc     768
   Ser Pro Ser Phe Ile Gln Phe His Pro Thr Gly Leu Pro Val Asn Ser
                   245                 250                 255
   acc tgg cag tcc aag acc att ctg atg tcc gag tcg ctg cgt aac gac     816
   Thr Trp Gln Ser Lys Thr Ile Leu Met Ser Glu Ser Leu Arg Asn Asp
               260                 265                 270
   ggc cgc atc tgg tcc cct aag gaa ccg aac gat aac cgc gat cca aac     864
   Gly Arg Ile Trp Ser Pro Lys Glu Pro Asn Asp Asn Arg Asp Pro Asn
           275                 280                 285
   acc atc cct gag gat gag cgc gac tac ttc ctg gag cgc cgc tac cca     912
   Thr Ile Pro Glu Asp Glu Arg Asp Tyr Phe Leu Glu Arg Arg Tyr Pro
       290                 295                 300
   gca ttc ggt aac ctc gtc cca cgt gac gtt gct tcc cgt gcg atc tcc     960
   Ala Phe Gly Asn Leu Val Pro Arg Asp Val Ala Ser Arg Ala Ile Ser
   305                 310                 315                 320
   cag cag atc aat gct ggt ctc ggt gtt gga cct ctg aac aac gct gca    1008
   Gln Gln Ile Asn Ala Gly Leu Gly Val Gly Pro Leu Asn Asn Ala Ala
                   325                 330                 335
   tac ctg gac ttc cgc gac gcc acc gag cgc ctc gga cag gac acc atc     1056
   Tyr Leu Asp Phe Arg Asp Ala Thr Glu Arg Leu Gly Gln Asp Thr Ile
               340                 345                 350
   cgc gag cgt tac tcc aac ctc ttc acc atg tac gaa gag gca att ggc     1104
   Arg Glu Arg Tyr Ser Asn Leu Phe Thr Met Tyr Glu Glu Ala Ile Gly
           355                 360                 365
   gag gac cca tac tcc agc cca atg cgt att gca ccg acc tgc cac ttc     1152
   Glu Asp Pro Tyr Ser Ser Pro Met Arg Ile Ala Pro Thr Cys His Phe
       370                 375                 380
   acc atg ggt ggc ctc tgg act gac ttc aac gaa atg acg tca ctc cca     1200
   Thr Met Gly Gly Leu Trp Thr Asp Phe Asn Glu Met Thr Ser Leu Pro
   385                 390                 395                 400
   ggt ctg ttc tgc gca ggc gaa gca tcc tgg acc tac cac ggt gca aac     1248
   Gly Leu Phe Cys Ala Gly Glu Ala Ser Trp Thr Tyr His Gly Ala Asn
                   405                 410                 415
   cgt ctg ggc gca aac tcc ctg ctc tcc gct tcc gtc gat ggc tgg ttc     1296
   Arg Leu Gly Ala Asn Ser Leu Leu Ser Ala Ser Val Asp Gly Trp Phe
               420                 425                 430
   acc ctg cca ttc acc atc cct aac tac ctc ggc cca ttg ctt ggc tcc     1344
   Thr Leu Pro Phe Thr Ile Pro Asn Tyr Leu Gly Pro Leu Leu Gly Ser
           435                 440                 445
   gag cgt ctg tca gag gat gca cca gaa gca cag gca gcg att gcg cgt     1392
   Glu Arg Leu Ser Glu Asp Ala Pro Glu Ala Gln Ala Ala Ile Ala Arg
       450                 455                 460
   gca cag gct cgc att gac cgc ctc atg ggc aac cgc cca gag tgg gtc     1440
   Ala Gln Ala Arg Ile Asp Arg Leu Met Gly Asn Arg Pro Glu Trp Val
   465                 470                 475                 480
   ggt gac aac gtt cac gga cct gag tac tac cac cgc cag ctt ggc gat     1488
   Gly Asp Asn Val His Gly Pro Glu Tyr Tyr His Arg Gln Leu Gly Asp
                   485                 490                 495
   atc ctg tac ttc tcc tgt ggc gtt tcc cga aac gta gaa gac ctc cag     1536
   Ile Leu Tyr Phe Ser Cys Gly Val Ser Arg Asn Val Glu Asp Leu Gln
               500                 505                 510
   gat ggc atc aac aag atc cgt gcc ctc cgc gat gac ttc tgg aag aac     1584
   Asp Gly Ile Asn Lys Ile Arg Ala Leu Arg Asp Asp Phe Trp Lys Asn
           515                 520                 525
   atg cgc atc acc ggc agc acc gat gag atg aac cag gtt ctc gaa tac     1632
   Met Arg Ile Thr Gly Ser Thr Asp Glu Met Asn Gln Val Leu Glu Tyr
       530                 535                 540
   gca gca cgc gta gcc gac tac atc gac ctc ggc gaa ctc atg tgt gtc     1680
   Ala Ala Arg Val Ala Asp Tyr Ile Asp Leu Gly Glu Leu Met Cys Val
   545                 550                 555                 560
   gac gcc ctc gac cgc gac gag tcc tgt ggc gct cac ttc cgc gac gac     1728
   Asp Ala Leu Asp Arg Asp Glu Ser Cys Gly Ala His Phe Arg Asp Asp
                   565                 570                 575
   cac ctc tcc gaa gat ggc gaa gca gaa cgt gac gac gaa aac tgg tgc     1776
   His Leu Ser Glu Asp Gly Glu Ala Glu Arg Asp Asp Glu Asn Trp Cys
               580                 585                 590
   ttc gtc tcc gca tgg gaa cca ggc gag aac gga acc ttc gtc cgc cac     1824
   Phe Val Ser Ala Trp Glu Pro Gly Glu Asn Gly Thr Phe Val Arg His
           595                 600                 605
   gca gaa cca ctg ttc ttc gaa tcc gtc cca ctg cag aca agg aac tac     1872
   Ala Glu Pro Leu Phe Phe Glu Ser Val Pro Leu Gln Thr Arg Asn Tyr
       610                 615                 620
   aag taa                                                             1878
   Lys
   625
<210> 5
   <211> 625
   <212> PRT
   <213> Corynebacterium glutamicum
<400> 5
   Met Asn Leu Val Ser Pro Leu Asn Arg Arg Lys Phe Arg Val Leu Val
   1               5                   10                  15
   Val Gly Thr Gly Leu Ser Gly Gly Ala Ala Ala Ala Ala Leu Gly Glu
               20                  25                  30
   Leu Gly Tyr Asp Val Lys Ala Phe Thr Tyr His Asp Ala Pro Arg Arg
           35                  40                  45
   Ala His Ser Ile Ala Ala Gln Gly Gly Val Asn Ser Ala Arg Gly Lys
       50                  55                  60
   Lys Val Asp Asn Asp Gly Ala Tyr Arg His Val Lys Asp Thr Val Lys
   65                  70                  75                  80
   Gly Gly Asp Tyr Arg Gly Arg Glu Ser Asp Cys Trp Arg Leu Ala Val
                   85                  90                  95
   Glu Ser Val Arg Val Ile Asp His Met Asn Ala Ile Gly Ala Pro Phe
               100                 105                 110
   Ala Arg Glu Tyr Gly Gly Ala Leu Ala Thr Arg Ser Phe Gly Gly Val
           115                 120                 125
   Gln Val Ser Arg Thr Tyr Tyr Thr Arg Gly Gln Thr Gly Gln Gln Leu
       130                 135                 140
   Gln Leu Ser Thr Ala Ser Ala Leu Gln Arg Gln Ile His Leu Gly Ser
   145                 150                 155                 160
   Val Glu Ile Phe Thr His Asn Glu Met Val Asp Val Ile Val Thr Glu
                   165                 170                 175
   Arg Asn Gly Glu Lys Arg Cys Glu Gly Leu Ile Met Arg Asn Leu Ile
               180                 185                 190
   Thr Gly Glu Leu Thr Ala His Thr Gly His Ala Val Ile Leu Ala Thr
           195                 200                 205
   Gly Gly Tyr Gly Asn Val Tyr His Met Ser Thr Leu Ala Lys Asn Ser
       210                 215                 220
   Asn Ala Ser Ala Ile Met Arg Ala Tyr Glu Ala Gly Ala Tyr Phe Ala
   225                 230                 235                 240
   Ser Pro Ser Phe Ile Gln Phe His Pro Thr Gly Leu Pro Val Asn Ser
                   245                 250                 255
   Thr Trp Gln Ser Lys Thr Ile Leu Met Ser Glu Ser Leu Arg Asn Asp
               260                 265                 270
   Gly Arg Ile Trp Ser Pro Lys Glu Pro Asn Asp Asn Arg Asp Pro Asn
           275                 280                 285
   Thr Ile Pro Glu Asp Glu Arg Asp Tyr Phe Leu Glu Arg Arg Tyr Pro
       290                 295                 300
   Ala Phe Gly Asn Leu Val Pro Arg Asp Val Ala Ser Arg Ala Ile Ser
   305                 310                 315                 320
   Gln Gln Ile Asn Ala Gly Leu Gly Val Gly Pro Leu Asn Asn Ala Ala
                   325                 330                 335
   Tyr Leu Asp Phe Arg Asp Ala Thr Glu Arg Leu Gly Gln Asp Thr Ile
               340                 345                 350
   Arg Glu Arg Tyr Ser Asn Leu Phe Thr Met Tyr Glu Glu Ala Ile Gly
           355                 360                 365
   Glu Asp Pro Tyr Ser Ser Pro Met Arg Ile Ala Pro Thr Cys His Phe
       370                 375                 380
   Thr Met Gly Gly Leu Trp Thr Asp Phe Asn Glu Met Thr Ser Leu Pro
   385                 390                 395                 400
   Gly Leu Phe Cys Ala Gly Glu Ala Ser Trp Thr Tyr His Gly Ala Asn
                   405                 410                 415
   Arg Leu Gly Ala Asn Ser Leu Leu Ser Ala Ser Val Asp Gly Trp Phe
               420                 425                 430
   Thr Leu Pro Phe Thr Ile Pro Asn Tyr Leu Gly Pro Leu Leu Gly Ser
           435                 440                 445
   Glu Arg Leu Ser Glu Asp Ala Pro Glu Ala Gln Ala Ala Ile Ala Arg
       450                 455                 460
   Ala Gln Ala Arg Ile Asp Arg Leu Met Gly Asn Arg Pro Glu Trp Val
   465                 470                 475                 480
   Gly Asp Asn Val His Gly Pro Glu Tyr Tyr His Arg Gln Leu Gly Asp
                   485                 490                 495
   Ile Leu Tyr Phe Ser Cys Gly Val Ser Arg Asn Val Glu Asp Leu Gln
               500                 505                 510
   Asp Gly Ile Asn Lys Ile Arg Ala Leu Arg Asp Asp Phe Trp Lys Asn
           515                 520                 525
   Met Arg Ile Thr Gly Ser Thr Asp Glu Met Asn Gln Val Leu Glu Tyr
       530                 535                 540
   Ala Ala Arg Val Ala Asp Tyr Ile Asp Leu Gly Glu Leu Met Cys Val
   545                 550                 555                 560
   Asp Ala Leu Asp Arg Asp Glu Ser Cys Gly Ala His Phe Arg Asp Asp
                   565                 570                 575
   His Leu Ser Glu Asp Gly Glu Ala Glu Arg Asp Asp Glu Asn Trp Cys
               580                 585                 590
   Phe Val Ser Ala Trp Glu Pro Gly Glu Asn Gly Thr Phe Val Arg His
           595                 600                 605
   Ala Glu Pro Leu Phe Phe Glu Ser Val Pro Leu Gln Thr Arg Asn Tyr
       610                 615                 620
   Lys
   625
<220> 6
   <211> 855
   <212> DNA
   <213> Corynebacterium glutamicum
<220>
   <221> CDS
   <222> (1)..(852)
   <223> sdhB
<400> 6
<210> 7
   <211> 284
   <212> PRT
   <213> Corynebacterium glutamicum
<400> 7
   Val Leu Arg Leu Arg Met Gly Thr Arg Arg Glu Arg Asn Leu Arg Pro
   1               5                   10                  15
   Pro Arg Arg Thr Thr Val Leu Arg Ile Arg Pro Thr Ala Asp Lys Glu
               20                  25                  30
   Leu Gln Val Met Lys Leu Thr Leu Glu Ile Trp Arg Gln Ala Gly Pro
           35                  40                  45
   Thr Ala Glu Gly Lys Phe Glu Thr Val Gln Val Asp Asp Ala Val Ala
       50                  55                  60
   Gln Met Ser Ile Leu Glu Leu Leu Asp His Val Asn Asn Lys Phe Ile
   65                  70                  75                  80
   Glu Glu Gly Lys Glu Pro Phe Ala Phe Ala Ser Asp Cys Arg Glu Gly
                   85                  90                  95
   Ile Cys Gly Thr Cys Gly Leu Leu Val Asn Gly Arg Pro His Gly Ala
               100                 105                 110
   Asp Gln Asn Lys Pro Ala Cys Ala Gln Arg Leu Val Ser Tyr Lys Glu
           115                 120                 125
   Gly Asp Thr Leu Lys Ile Glu Pro Leu Arg Ser Ala Ala Tyr Pro Val
       130                 135                 140
   Ile Lys Asp Met Val Val Asp Arg Ser Ala Leu Asp Arg Val Met Glu
   145                 150                 155                 160
   Gln Gly Gly Tyr Val Thr Ile Asn Ala Gly Thr Ala Pro Asp Ala Asp
                   165                 170                 175
   Thr Leu His Val Asn His Glu Thr Ala Glu Leu Ala Leu Asp His Ala
               180                 185                 190
   Ala Cys Ile Gly Cys Gly Ala Cys Val Ala Ala Cys Pro Asn Gly Ala
           195                 200                 205
   Ala His Leu Phe Thr Gly Ala Lys Leu Val His Leu Ser Leu Leu Pro
       210                 215                 220
   Leu Gly Lys Glu Glu Arg Gly Leu Arg Ala Arg Lys Met Val Asp Glu
   225                 230                 235                 240
   Met Glu Thr Asn Phe Gly His Cys Ser Leu Tyr Gly Glu Cys Ala Asp
                   245                 250                 255
   Val Cys Pro Ala Gly Ile Pro Leu Thr Ala Val Ala Ala Val Thr Lys
               260                 265                 270
   Glu Arg Ala Arg Ala Ala Phe Arg Gly Lys Asp Asp
           275                 280

## Beschreibung

Gegenstand der Erfindung sind für die Gene sdhC, sdhA und sdhB codierende Nukleotidsequenzen aus coryneformen Bakterien und ein Verfahren zur fermentativen Herstellung von L-Aminosäuren, insbesondere L-Lysin, durch Abschwächung des sdhC- und/oder des sdhA- und/oder des sdhB-Gens.

### Stand der Technik

L-Aminosäuren, insbesondere Lysin, finden in der Humanmedizin und in der pharmazeutischen Industrie, in der Lebensmittelindustrie und ganz besonders in der Tierernährung Anwendung.

Es ist bekannt, daß L-Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff oder die Zusammensetzung der Nährmedien wie z.B. die Zuckerkonzentration während der Fermentation oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die L-Aminosäuren produzieren.

Seit einigen Jahren werden ebenfalls Methoden der rekombinanten DNA-Technik zur Stammverbesserung von L-Aminosäure produzierenden Stämmen von Corynebacterium eingesetzt.

### Aufgabe der Erfindung

Die Erfinder haben sich zur Aufgabe gestellt, neue Maßnahmen zur verbesserten fermentativen Herstellung von Aminosäuren, insbesondere L-Lysin, bereitzustellen.

### Beschreibung der Erfindung

L-Aminosäuren, insbesondere Lysin, finden in der Humanmedizin und in der pharmazeutischen Industrie, in der Lebensmittelindustrie und ganz besonders in der Tierernährung Anwendung. Es besteht daher ein allgemeines Interesse daran, neue verbesserte Verfahren zur Herstellung von L-Aminosäuren, insbesondere L-Lysin, bereitzustellen.

Gegenstand der Erfindung ist ein isoliertes Polynukleotid enthaltend eine Polynukleotidsequenz, ausgewählt aus der Gruppe
a) Polynukleotid, das für ein Polypeptid codiert, das die Aminosäuresequenz aus der SEQ ID No. 3 enthält,
b) Polynukleotid, das für ein Polypeptid codiert, das die Aminosäuresequenz von SEQ ID No. 5 enthält,
c) Polynukleotid, das für ein Polypeptid codiert, das die Aminosäuresequenz von SEQ ID No. 7 enthält,
d) Polynukleotid, das für ein Polypeptid codiert, das eine Aminosäuresequenz enthält, die zu mindestens 90% identisch ist mit der Aminosäuresequenz von SEQ ID No. 3,
e) Polynukleotid, das für ein Polypeptid codiert, das eine Aminosäuresequenz enthält, die zu mindestens 90% identisch ist mit der Aminosäuresequenz von SEQ ID No. 7,
g) Polynukleotid, das komplementär ist zu den Polynukleotiden von a), b), c), d), oder e).

Gegenstand der Erfindung ist ebenfalls ein Polynukleotid, das eine in coryneformen Bakterien replizierbare rekombinante DNA ist.

Gegenstand der Erfindung ist ebenfalls ein Polynukleotid, das eine RNA ist.

Gegenstand der Erfindung sind ebenso Polynukleotide, ausgewählt sind aus der Gruppe:
a) Polynukleotid, enthaltend eine Nukleotidsequenz, ausgewählt aus SEQ ID NO:1, SEQ ID NO:2, SEQ ID NO:4 oder SEQ ID NO:6,
a) Polynukleotid, enthaltend eine Nukleotidsequenz, die der SEQ ID NO:1, SEQ ID NO:2, SEQ ID NO:4 oder SEQ ID NO:6 innerhalb des Bereichs der Degeneration des genetischen Codes entspricht,
b) Polynukleotid, enthaltend eine Nukleotidsequenz mit funktionsneutralen Sinnmutanten in SEQ ID NO:1, SEQ ID NO:2, SEQ ID NO:4 oder SEQ ID NO: 6.

Teil der Erfindung sind auch Polypeptid-Untereinheiten A, B und C, die in ihrer Gesamtwirkung die Aktivität einer Succinat-Dehydrogenase besitzen, wobei die Aminosäuresequenzen der Polypeptid-Untereinheiten B und C zu mindestens 90 % identisch sind mit den Aminosäuresequenzen enthalten in
a) SEQ ID NO:3 (Untereinheit C)
b) SEQ ID NO:7 (Untereinheit B),
und die Aminosäuresequenz der Polypeptiduntereinheit A identisch ist mit der Aminosäuresequenz enthalten in SEQ ID NO:5.

Ebenso ein isoliertes coryneformes Bakterium, in dem die Expression eines oder mehrerer der Polynukleotide ausgeschaltet wird, die für die Polypeptid-Untereinheiten A, B und C kodieren, die in ihrer Gesamtwirkung die Aktivität einer Succinat-Dehydrogenase besitzen, wobei die Aminosäuresequenzen der Untereinheiten B und C zu mindestens 90 % identisch sind mit den Aminosäuresequenzen enthalten in
a) SEQ ID NO:3 (Untereinheit C), und
b) SEQ ID NO:7 (Untereinheit B)
und die Aminosäuresequenz der Polypeptiduntereinheit A identisch ist mit der Aminosäuresequenz enthalten in SEQ ID NO:5.

Beansprucht werden auch coryneforme Bakterien, die eine Deletion, Insertion oder einen Basenaustausch in mindestens einer der Untereinheiten A, B oder C enthalten.

Weitere Gegenstände sind ein Vektor, enthaltend eines der genannten Polynukleotide und als Wirtszelle dienende coryneforme Bakterien, die den Vektor enthalten.

Beschrieben werden ebenso Polynukleotide, die im wesentlichen aus einer Polynukleotidsequenz bestehen, die erhältlich sind durch Screening mittels Hybridisierung einer entsprechenden Genbank, die das vollständige sdhC- und/oder sdhA und/oder sdhB-Gen mit der Polynukleotidsequenz entsprechend SEQ ID No. 1 enthalten mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Beschreibung sind geeignet als Hybridisierungs-Sonden für RNA, cDNA und DNA, um cDNA in voller Länge zu isolieren, die für Succinate-Dehydrogenase oder deren Untereinheiten A, B oder C codieren und solche cDNA oder Gene zu isolieren, die eine hohe Ähnlichkeit der Sequenz mit der der Gene für die Succinate-Dehydrogenase oder deren Untereinheiten A, B oder C aufweisen.

Die beschriebenen Polynukleotidsequenzen sind weiterhin als Primer geeignet, mit deren Hilfe mit der Polymerase-Kettenreaktion (PCR) DNA von Genen hergestellt werden kann, die für Succinate-Dehydrogenase codieren.

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen die Polypeptide gemäß SEQ ID No. 3 und gemäß SEQ ID No. 7, insbesondere solche mit der biologischen Aktivität der Succinate-Dehydrogenase und auch solche ein, die zu wenigstens 90% identisch sind mit dem Polypeptid gemäß SEQ ID No. 3 und SEQ ID No. 7, besonders zu wenigstens 90 % bis 95 % Identität mit dem Polypeptid gemäß SEQ ID No. 3 und gemäß SEQ ID No. 7 und die genannte Aktivität aufweisen.

Die Erfindung betrifft weiterhin ein Verfahren zur fermentativen Herstellung von L-Aminosäuren, insbesondere Lysin, unter Verwendung von coryneformen Bakterien, die insbesondere bereits die L-Aminosäuren insbesondere L-Lysin produzieren und in denen die für das sdhC-Gen und/oder das sdhA-Gen und/oder das sdhB-Gen codierenden Nukleotidsequenzen abgeschwächt, insbesondere auf niedrigem Niveau exprimiert werden.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA codiert werden, indem man beispielsweise einen schwachen Promotor verwendet oder ein Gen bzw. Allel verwendet, das für ein entsprechendes Enzym mit einer niedrigen Aktivität codiert bzw. das entsprechende Gen oder Enzym (Protein) inaktiviert und gegebenenfalls diese Maßnahmen kombiniert.

Die Mikroorganismen, die Gegenstand der vorliegenden Erfindung sind, können L-Aminosäuren, insbesondere L-Lysin, aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme,

wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM 5714

Den Erfindern gelang es, die neuen, für das Enzym Succinat-Dehydrogenase (EC 1.3.99.1) codierenden Gene sdhC, sdhA und sdhB von C. glutamicum zu isolieren.

Zur Isolierung des sdhC- und/oder des sdhA-Gens und/oder des sdhB-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). O'Donohue (The Cloning and Molecular Analysis of Four Common Aromatic Amino Acid Biosynthetic Genes from Corynebacterium glutamicum. Ph.D. Thesis, National University of Ireland, Galway, 1997) beschreibt die Klonierung von C. glutamicum Genen unter Verwendung des von Short et al. (Nucleic Acids Research, 16: 7583) beschriebenen λ Zap Expressionssystems.

Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind wie beispielsweise der Stamm DH5a (Jeffrey H. Miller: "A Short Course in Bacterial Genetics, A Laboratory Manual and Handbook for Escherichia coli and Related Bacteria", Cold Spring Harbour Laboratory Press, 1992).

Die mit Hilfe von Cosmiden oder anderen λ-Vektoren klonierten langen DNA-Fragmente können anschließend wiederum in gängige, für die DNA-Sequenzierung geeignete Vektoren subkloniert werden.

Methoden zur DNA-Sequenzierung sind unter anderem bei Sanger et al. (Proceedings of the National Academy of Sciences of the United States of America USA, 74:5463-5467, 1977) beschrieben.

Die erhaltenen DNA-Sequenzen können dann mit bekannten Algorithmen bzw. Sequenzanalyse-Programmen wie z.B. dem von Staden (Nucleic Acids Research 14, 217-232(1986)), dem GCG-Programm von Butler (Methods of Biochemical Analysis 39, 74-97 (1998)) dem FASTA-Algorithmus von Pearson und Lipman (Proceedings of the National Academy of Sciences USA 85,2444-2448 (1988)) oder dem BLAST-Algorithmus von Altschul et al. (Nature Genetics 6, 119-129 (1994)) untersucht und mit den in öffentlich zugänglichen Datenbanken vorhandenen Sequenzeinträgen verglichen werden. Öffentlich zugängliche Datenbanken für Nukleotidsequenzen sind beispielsweise die der European Molecular Biologies Laboratories (EMBL, Heidelberg, Deutschland) oder die des National Center for Biotechnology Information (NCBI, Bethesda, MD, USA).

Auf diese Weise wurde die neue für das sdhC-Gen und das sdhA-Gen und das sdhB-Gen codierende DNA-Sequenz von C. glutamicum erhalten, die als SEQ ID No. 1 Bestandteil der vorliegenden Erfindung ist. Weiterhin wurde aus der vorliegenden DNA-Sequenz mit den oben beschriebenen Methoden die Aminosäuresequenz der entsprechenden Proteine abgeleitet. In SEQ ID No. 3, SEQ ID No.5 und SEQ ID No. 7 sind die sich ergebenden Aminosäuresequenzen des sdhC, des sdhA und des sdhB Genproduktes dargestellt.

Codierende DNA-Sequenzen, die sich aus SEQ ID No. 1 durch die Degeneriertheit des genetischen Codes ergeben, sind ebenfalls Bestandteil der Erfindung. In der Fachwelt sind weiterhin konservative Aminosäureaustausche wie z. B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO. 1 ergeben, und diese Aminosäuresequenzen codierende DNA-Sequenzen sind ebenfalls Bestandteil der Erfindung.

In gleicher Weise sind DNA-Sequenzen, die mit SEQ ID No. 1 oder Teilen von SEQ ID No. 1 hybridisieren, Bestandteil der Beschreibung. Schließlich sind DNA-Sequenzen Bestandteil der Beschreibung, die durch die Polymerase-Kettenreaktion (PCR) unter Verwendung von Primern hergestellt werden, die sich aus SEQ ID No. 1 ergeben.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Abschwächung des sdhC- und/oder sdhA- und/oder des sdhB-Gens in verbesserter Weise L-Aminosäuren, insbesondere L-Lysin, produzieren.

Zur Erzielung einer Abschwächung können entweder die Expression des sdhC- und/oder sdhA- und/oder des sdhB-Gens oder die katalytischen Eigenschaften der Enzymproteine herabgesetzt oder ausgeschaltet werden. Gegebenenfalls können beide Maßnahmen kombiniert werden.

Die Erniedrigung der Genexpression kann durch geeignete Kulturführung oder durch genetische Veränderung (Mutation) der Signalstrukturen der Genexpression erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Patek et al. (Microbiology 142: 1297 (1996)) und in bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs,Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen (missense mutations) oder Nichtsinnmutationen (nonsense mutations) gesprochen. Insertionen oder Deletionen von mindestens einem Basenpaar in einem Gen führen zu Rasterverschiebungsmutationen (frame shift mutations), in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Eine gebräuchliche Methode, Gene von C. glutamicum zu mutieren, ist die von Schwarzer und Pühler (Bio/Technology 9, 84-87 (1991)) beschriebene Methode der Gen-Unterbrechung (gene disruption) und des Gen-Austauschs (gene replacement).

Bei der Methode der Gen-Unterbrechung wird ein zentraler Teil der Codierregion des interessierenden Gens in einen Plasmidvektor kloniert, der in einem Wirt (typischerweise E. coli), nicht aber in C. glutamicum replizieren kann. Als Vektoren kommen bespielsweise pSUP301 (Simon et al., Bio/Technology 1, 784-791 (1983)), pK18mob oder pK19mob (Schäfer et al., Gene 145, 69-73 (1994)), pK18mobsacB oder pK19mobsacB (Jäger et al., Journal of Bacteriology 174: 5462-65 (1992)), pGEM-T (Promega corporation, Madison, WI, USA), pCR2.1-TOPO (Shuman (1994). Journal of Biological Chemistry 269:32678-84; US-Patent 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zentrale Teil der Codierregion des Gens enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses wird die Codierregion des betreffenden Gens durch die Vektorsequenz unterbrochen und man erhält zwei unvollständige Allele, denen jeweils das 3'- bzw. das 5'-Ende fehlt. Diese Methode wurde beispielsweise von Fitzpatrick et al. (Applied Microbiology and Biotechnology 42, 575-580 (1994)) zur Ausschaltung des recA-Gens von C. glutamicum verwendet. Das sdhC- und/oder das sdhA- und/oder das sdhB-Gen können auf diese Weise ausgeschaltet werden.

Bei der Methode des Genaustaussches (gene replacement) wird eine Mutation wie z.B. eine Deletion, Insertion oder Basenaustausch in dem interessierenden Gen in-vitro hergestellt. Das hergestellte Allel wird wiederum in einen für C. glutamicum nicht replikativen Vektor kloniert und dieser anschließend durch Transformation oder Konjugation in den gewünschten Wirt von C. glutamicum überführt. Nach homologer Rekombination mittels eines ersten, Integration bewirkenden "cross over"-Ereignisses und eines geeigneten zweiten, eine Excision bewirkenden "cross over"-Ereignisses im Zielgen bzw. in der Zielsequenz erreicht man den Einbau der Mutation bzw. des Allels. Diese Methode wurde beispielsweise von Peters-Wendisch (Microbiology 144, 915 - 927 (1998)) verwendet, um das pyc-Gen von C. glutamicum durch eine Deletion auszuschalten. In das sdhC- und/oder das sdhA- und/oder das sdhB-Gen kann auf diese Weise eine Deletion, Insertion oder ein Basenaustausch eingebaut werden.

Ein weiterer Gegenstand der Erfindung ist demnach ein Verfahren zur fermentativen Herstellung von L-Aminosäuren, insbesondere L-Lysin, bei dem man entweder einen mit einem Plasmidvektor transformierten Stamm einsetzt, und der Plasmidvektor Nukleotidsequenzen der für das Enzym Succinat-Dehydrogenase codierenden Gene trägt oder der Stamm eine Deletion, Insertion oder einen Basenaustausch im sdhC- und/oder sdhA- und/oder sdhB-Gen trägt.

Verfahren zur fermentativen Herstellung von L-Aminosäuren, insbesondere L-Lysin, enthalten folgende Schritte:
a) Fermentation von die L-Aminosäure produzierenden coryneformen Bakterien, in denen man zumindest eines der Gene,ausgewählt aus den für das Enzym Succinate-Dehydrogenase und dessen Untereinheiten A, B und C codierenden Genen, abschwächt,
b) Anreicherung der L-Aminosäure im Medium oder in den Zellen der Bakterien und
c) Isolieren der L-Aminosäure.

Zusätzlich kann es für die Produktion von L-Aminosäuren, insbesondere L-Lysin, vorteilhaft sein, zusätzlich zur Abschwächung des sdhC- und/oder des sdhA- und/oder des sdhB-Gen eines oder mehrere Enzyme des jeweiligen Biosyntheseweges, der Glykolyse, der Anaplerotik, des Zitronensäure-Zyklus oder des Aminosäure-Exports zu verstärken, insbesondere zu überexprimieren.

So kann beispielsweise für die Herstellung von L-Lysin
- gleichzeitig das für die Dihydrodipicolinat-Synthase Codierende dapA-Gen (EP-B 0 197 335), oder
- gleichzeitig das für die Glyceraldehyd-3-Phosphat-Dehydrogenase codierende gap-Gen (Eikmanns (1992). Journal of Bacteriology 174:6076-6086), oder
- gleichzeitig das für die Pyruvat-Carboxylase codierende pyc-Gen(DE-A-198 31 609), oder
- gleichzeitig das für die Malat-Chinon-Oxidoreduktase codierende mqo-Gen (Molenaar et al., European Journal of Biochemistry 254, 395-403 (1998)), oder
- gleichzeitig das für den Lysin-Export codierende lysE-Gen (DE-A-195 48 222)
überexprimiert werden.

Weiterhin kann es für die Produktion von L-Aminosäuren, insbesondere L-Lysin, vorteilhaft sein, neben den beanspruchten Genen gleichzeitig
- das für die Phosphoenolpyruvat-Carboxykinase codierende pck-Gen (DE 199 50 409.1, DSM 13047) und/oder
- das für die Glucose-6-Phosphat-Isomerase codierende pgi-Gen (US 09/396,478, DSM 12969)
abzuschwächen.

Weiterhin kann es für die Produktion von L-Aminosäuren, insbesondere L-Lysin, vorteilhaft sein, neben der Abschwächung des sdhC- und/oder des sdhA- und/oder des sdhB-Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die das Polynukleotid gemäß Anspruch 1 enthaltenden Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren, insbesondere L-Lysin, kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnußöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCosl (Wahl et al. (1987) Proceedings of the National Academy of Sciences USA 84:2160-2164), bezogen von der Firma Stratagene (La Jolla, USA, Produktbeschreibung SuperCosl Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Res. 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A laboratory Manual, Cold Spring Harbor) beschrieben durchgeführt, wobei die Zellen auf LB-Agar (Lennox, 1955, Virology, 1:190) mit 100 ug/ml Ampicillin ausplattiert wurden. Nach Inkubation über Nacht bei 37°C wurden rekombinante Einzelklone selektioniert.

### Beispiel 2

### Isolierung und Sequenzierung der Gene sdhC, sdhA und sdhB

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 ug/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academies of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZerol-Derivate wurden zu einem zusammenhängenden Contig assembliert. Die computergestützte Codierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID No. 1 dargestellt. Die Analyse der Nukleotidsequenz ergab ein offenes Leseraster von 879 Basenpaaren, welches als sdhC-Gen bezeichnet wurde, sowie ein offenes Leseraster von 1875 Basenparen, welches als sdhA bezeichnet wurde, sowie ein offenes Leseraster von 852 Basenpaaren, welches als sdhB-Gen bezeichnet wurde. Das sdhC-Gen codiert für ein Polypeptid von 293 Aminosäuren, welches in SEQ ID No. 3 dargestellt ist. Das sdhA-Gen codiert für ein Polypeptid von 625 Aminosäuren, welches in SEQ ID No. 5 dargestellt ist. Das sdhB-Gen codiert für ein Polypeptid von 284 Aminosäuren, welches in SEQ ID No. 7 dargestellt ist.

### Beispiel 3

### Herstellung eines Integrationsvektors für die Integrationsmutagenese des sdhA-Gens

Aus dem Stamm ATCC 13032 wurde nach der Methode von Eikmanns et al. (Microbiology 140: 1817 - 1828 (1994)) chromosomale DNA isoliert. Aufgrund der aus Beispiel 2 für C. glutamicum bekannten Sequenz des sdhA-Gens wurden die folgenden Oligonukleotide für die Polymerase Kettenreaktion ausgewählt:

Die dargestellten Primer wurden von der Firma MWG Biotech (Ebersberg, Deutschland) synthetisiert und nach der Standard-PCR-Methode von Innis et al. (PCR protocols. A guide to methods and applications, 1990, Academic Press) mit der Pwo-Polymerase der Firma Boehringer Mannheim (Deutschland, Produktbeschreibung Pwo DNA Polymerase, Product No. 1 644 947) die PCR Reaktion durchgeführt. Mit Hilfe der Polymerase-Kettenreaktion ermöglichen die Primer die Amplifikation eines ca. 0,67 kb großen internen Fragmentes des sdhA-Gens. Das so amplifizierte Produkt wurde in einem 0,8%igen Agarosegel elektrophoretisch geprüft.

Das amplifizierte DNA Fragment wurde mit dem Zero Blunt^{™} Kit der Firma Invitrogen Corporation(Carlsbad, CA, USA; Katalog Nummer K2700-20) in den Vektor pCR®Blunt II (Bernard et al., Journal of Molecular Biology, 234: 534-541 (1993)) ligiert.

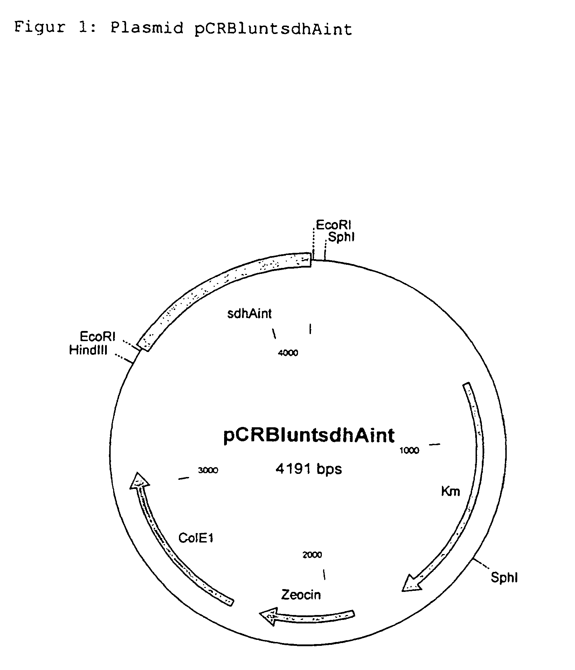

Anschließend wurde der E. coli Stamm TOP10 mit dem Ligationsansatz (Hanahan, In: DNA cloning. A practical approach. Vol.I. IRL-Press, Oxford, Washington DC, USA, 1985) elektroporiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y., 1989), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCRBluntsdhAint genannt und ist in Figur 1 dargestellt.

### Beispiel 4

### Integrationsmutagenese des sdhA-Gens in dem Stamm DSM 5715

Der in Beispiel 3 genannte Vektor pCRBluntsdhAint wurde nach der Elektroporationsmethode von Tauch et.al.(FEMS Microbiological Letters, 123:343-347 (1994)) in Corynebacterium glutamicum DSM 5715 elektroporiert. Der Stamm DSM 5715 ist in der EP-B-0435132 beschrieben. Der Vektor pCRBluntsdhAint kann in DSM5715 nicht selbständig replizieren und bleibt nur dann in der Zelle erhalten, wenn er ins Chromosom von DSM 5715 integriert hat. Die Selektion von Klonen mit ins Chromosom integriertem pCRBluntsdhAint erfolgte durch Ausplattieren des Elektroporationsansatzes auf LB Agar (Sambrook et al., Molecular cloning: a laboratory manual. 2^{nd} Ed. Cold Spring Harbor Laboratory Press, Cold Spring Harbor, N.Y.), der mit 15 mg/l Kanamycin supplementiert worden war.

Für den Nachweis der Integration wurde das sdhAint-Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert. Chromosomale DNA eines potentiellen Integranten wurde nach der Methode von Eikmanns et al. (Microbiology 140: 1817 - 1828 (1994)) isoliert und jeweils mit den Restriktionsenzymen SphI und HindIII geschnitten. Die entstehenden Fragmente wurden mittels der Agarosegel-Elektrophorese aufgetrennt und mit dem Dig-Hybrisierungskit der Firma Boehringer bei 68°C hybridisiert. Das in Beispiel 3 genannte Plasmid pCRBluntsdhAint hatte innerhalb des chromosomalen sdhA-Gens ins Chromosom von DSM5715 inseriert. Der Stamm wurde als DSM5715::pCRBluntsdhAint bezeichnet.

### Beispiel 5

### Herstellung von L-Glutaminsäure mit dem Stamm DSM5715::pCRBluntsdhAint

Der in Beispiel 4 erhaltene C. glutamicum Stamm DSM5715::pCRBluntsdhAint wurde in einem zur Produktion von Glutaminsäure geeigneten Nährmedium kultiviert und der Glutaminsäuregehalt im Kulturüberstand bestimmt.

Dazu wurde der Stamm zunächst auf Agarplatte mit dem entsprechenden Antibiotikum (Hirn-Herz Agar mit Kanamycin (25 mg/1) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet.

| | |
|---|---|
| Medium Cg III | |
| NaCl | 2,5 g/l |
| Bacto-Pepton | 10 g/l |
| Bacto-Yeast-Extrakt | 10 g/l |
| Glucose (getrennt autoklaviert) | 2% (w/v) |

Der pH-Wert wurde auf pH 7.4 eingestellt

Diesem wurde Kanamycin (25 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 OD betrug. Für die Hauptkultur wurde das Medium MM verwendet.

| | |
|---|---|
| Medium MM | |
| CSL (Corn Steep Liquor) | 5 g/l |
| MOPS (Morpholinopropansufonsäure) | 20 g/l |
| Natriumacetat (sterilfiltriert) | 20g/l |
| Salze: | |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0 mg/l |
| Biotin (sterilfiltriert) | 0,3 mg/l |
| Thiamin * HCl (sterilfiltriert) | 0,2 mg/l |
| Leucin (sterilfiltriert) | 0,1 g/l |
| CaCO₃ | 25 g/l |

CSL, MOPS und die Salzlösung werden mit Ammoniakwasser auf pH 7 eingestellt und autoklaviert. Anschließend werden die sterilen Substrat- und Vitaminlösungen zugesetzt, sowie das trocken autoklavierte CaCO₃ zugesetzt.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchtigkeit.

Nach 24 Stunden wurde die OD bei einer Meßwellenlänge von 660 nm mit dem Biomek 1000 (Beckmann Instruments GmbH, München) ermittelt. Die gebildete Glutaminsäuremenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | L-Glutaminsäure (mg/l) |
|---|---|---|
| DSM5715 | 6,6 | 41 |
| DSM5715::pCRBluntsdhAint | 5,1 | 155 |

Folgende Figuren sind beigefügt:
Figur 1: Karte des Plasmids pCRBluntsdhAint

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.

| | |
|---|---|
| Km: | Kanamycin Resistenz-Gen |
| Zeocin: | Zeocin Resistenz-Gen |
| HindIII: | Schnittstelle des Restriktionsenzyms HindIII |
| SphI: | Schnittstelle des Restriktionsenzyms SphI |
| EcoRI: | Schnittstelle des Restriktionsenzyms EcoRI |
| sdhAint: | internes Fragment des sdhA-Gens |
| ColE1 ori: | Replikationsursprung des Plasmides ColE1 |

## Patentansprüche

1. Isoliertes Polynukleotid aus coryneformen Bakterien, enthaltend eine Polynukleotidsequenz, ausgewählt aus der Gruppe
a) Polynukleotid, das für ein Polypeptid codiert, das die Aminosäuresequenz aus der SEQ ID No. 3 enthält,
b) Polynukleotid, das für ein Polypeptid codiert, das die Aminosäuresequenz von SEQ ID No. 5 enthält,
c) Polynukleotid, das für ein Polypeptid codiert, das die Aminosäuresequenz von SEQ ID No. 7 enthält,
d) Polynukleotid, das für ein Polypeptid codiert, das eine Aminosäuresequenz enthält, die zu mindestens 90% identisch ist mit der Aminosäuresequenz von SEQ ID No. 3,
e) Polynukleotid, das für ein Polypeptid codiert, das eine Aminosäuresequenz enthält, die zu mindestens 90% identisch ist mit der Aminosäuresequenz von SEQ ID No. 7
wobei die Polypeptide gemäss d) und e) zusammen mit dem Polypeptid gemäss b) in ihrer Gesamtwirkung die Aktivität einer Succinat-Dehydrogenase besitzen.
f) Polynukleotid, das komplementär ist zur den polynukleotiden von a), b), c), d) oder e),

2. Polynukleotid gemäß Anspruch 1,
wobei das Polynukleotid eine in coryneformen Bakterien rekombinante DNA ist.

3. Polynukleotid gemäß Anspruch 1,
wobei das Polynukleotid eine RNA ist.

4. Polynukleotid gemäß Anspruch 1, ausgewählt aus der Gruppe:
a) Polynukleotid, enthaltend eine Nukleotidsequenz, ausgewählt aus SEQ ID NO:1, SEQ ID NO:2, SEQ ID NO:4 oder SEQ ID NO:6,
a) Polynukleotid, enthaltend eine Nukleotidsequenz, die der SEQ ID NO:1, SEQ ID NO:2, SEQ ID NO:4 oder SEQ ID NO:6 innerhalb des Bereichs der Degeneration des genetischen Codes entspricht,
b) Polynukleotid, enthaltend eine Nukleotidsequenz mit funktionsneutralen Sinnmutanten in SEQ ID NO:1, SEQ ID NO:2, SEQ ID NO:4 oder SEQ ID NO:6.

5. Polypeptid-Untereinheiten A, B und C, die in ihrer Gesamtwirkung die Aktivität einer Succinat-Dehydrogenase besitzen, wobei die Aminosäuresequenzen der Polypeptid-Untereinheiten B und C zu mindestens 90 % identisch sind mit den Aminosäuresequenzen enthalten in
a) SEQ ID NO:3 (Untereinheit C)
b) SEQ ID NO:7 (Untereinheit B),
und die Aminosäuresequenz der Polypeptiduntereinheit A identisch ist mit der Aminosäuresequenz enthalten in SEQ ID NO:5.

6. Isoliertes coryneformes Bakterium, in dem die Expression eines oder mehrerer der Polynukleotide ausgeschaltet wird, die für die Polypeptid-Untereinheiten A, B und C kodieren, die in ihrer Gesamtwirkung die Aktivität einer Succinat-Dehydrogenase besitzen, wobei die Aminosäuresequenzen der Untereinheiten B und C zu mindestens 90 % identisch sind mit den Aminosäuresequenzen enthalten in
a) SEQ ID NO:3 (Untereinheit C), und
b) SEQ ID NO:7 (Untereinheit B)
und die Aminosäuresequenz der Polypeptiduntereinheit A identisch ist mit der Aminosäuresequenz enthalten in SEQ ID NO:5.

7. Coryneforme Bakterien gemäß Anspruch 6, die eine Deletion, Insertion oder einen Basenaustausch in mindestens einer der Untereinheiten A, B oder C enthalten.

8. Verfahren zur Herstellung von L-Aminosäuren, **dadurch gekennzeichnet, dass** man Bakterien gemäß den Ansprüchen 6 oder 7 in einem geeigneten Medium fermentiert.

9. Verfahren zur Herstellung von L-Aminosäuren gemäss Anspruch 8, bei dem man die L-Aminosäure im Medium oder in den Zellen anreichert und die L-Aminosäure isoliert.

10. Verfahren gemäß Anspruch 8, **dadurch gekennzeichnet, dass** man Bakterien einsetzt, in denen man zusätzlich weitere Gene des Biosyntheseweges der gewünschten L-Aminosäure verstärkt.

11. Verfahren gemäß Anspruch 8, **dadurch gekennzeichnet, dass** man Bakterien einsetzt, in denen die Stoffwechselwege zumindest teilweise ausgeschaltet sind, die die Bildung der gewünschten L-Aminosäure verringern.

12. Verfahren gemäß einem oder mehreren der Ansprüche 8 bis 11, **dadurch gekennzeichnet, dass** man L-Lysin oder L-Glutaminsäure herstellt.

13. Verfahren gemäß den Ansprüchen 8 - 12, **dadurch gekennzeichnet, dass** man Bakterien fermentiert, in denen man gleichzeitig eines oder mehrere der Gene, ausgewählt aus der Gruppe
a) das für die Dihydrodipicolinat-Synthase kodierende dapA-Gen,
b) das für die Glyceraldehyd-3-Phosphat Dehydrogenase kodierende gap-Gen,
c) das für die Pyruvat-Carboxylase kodierende pyc-Gen,
d) das für die Malat:Chinon Oxidoreduktase kodierende mqo-Gen,
e) das für ein Protein für den Lysin-Export kodierende lysE-Gen,
verstärkt insbesondere überexprimiert oder amplifiziert.

14. Verfahren gemäß den Ansprüchen 8 bis 13, **dadurch gekennzeichnet, dass** man Bakterien fermentiert, in denen man gleichzeitig eines oder mehrere der Gene, ausgewählt aus der Gruppe
a) das für die Phosphoenolpyruvat-Carboxykinase codierende pck-Gen
b) das für die Glucose-6-Phosphat Isomerase kodierende pgi-Gen
abschwächt.

15. Verfahren gemäss einem oder mehreren der vorhergehenden Ansprüche 8 bis 14, **dadurch gekennzeichnet, dass** man Mikroorganismen der Gattung Corynebacterium einsetzt.

16. Vektor enthaltend ein Polynukleotid gemäß Anspruch 1 a oder 1 d.

17. Vektor enthaltend ein Polynukleotid gemäß Anspruch 4.

18. Coryneforme Bakterien, enthaltend einen Vektor gemäß den Anspruchen 16 oder 17.

## Claims

1. Isolated polynucleotide from coryneform bacteria, comprising a polynucleotide sequence selected from the group consisting of
a) polynucleotide which encodes a polypeptide comprising the amino acid sequence from SEQ ID No. 3,
b) polynucleotide which encodes a polypeptide comprising the amino acid sequence of SEQ ID No. 5,
c) polynucleotide which encodes a polypeptide comprising the amino acid sequence from SEQ ID No. 7,
d) polynucleotide which encodes a polypeptide comprising an amino acid sequence which is at least 90% identical to the amino acid sequence of SEQ ID No. 3,
e) polynucleotide which encodes a polypeptide comprising an amino acid sequence which is at least 90% identical to the amino acid sequence of SEQ ID No. 7, the polypeptides according to d) and e), together with the polypeptide according to b), in its entirety has the activity of succinate-dehydrogenase,
f) polynucleotide which is complementary to the polynucleotides of a), b), c), d) or e).

2. Polynucleotide according to Claim 1, which is a DNA recombinant in coryneform bacteria.

3. Polynucleotide according to Claim 1, which is an RNA.

4. Polynucleotides according to Claim 1, selected from the group consisting of:
a) polynucleotide comprising a nucleotide sequence selected from SEQ ID NO:1, SEQ ID NO:2, SEQ ID NO:4 or SEQ ID NO:6,
b) polynucleotide comprising a nucleotide sequence which corresponds to SEQ ID NO:1, SEQ ID NO:2, SEQ ID NO:4 or SEQ ID NO:6 within the range of the degeneracy of the genetic code,
c) polynucleotide comprising a nucleotide sequence having functionally neutral sense mutants in SEQ ID NO:1, SEQ ID NO:2, SEQ ID NO:4 or SEQ ID NO:6.

5. Polypeptide subunits A, B and C whose overall action comprises the activity of a succinate dehydrogenase, the amino acid sequences of polypeptide subunits B and C being at least 90% identical to the amino acid sequences present in
a) SEQ ID NO:3 (subunit C)
b) SEQ ID NO:7 (subunit B),
and the amino acid sequence of polypeptide subunit A being identical to the amino acid sequence present in SEQ ID NO:5.

6. Isolated coryneform bacterium in which expression of one or more of the polynucleotides encoding polypeptide subunits A, B and C whose overall action comprises the activity of a succinate dehydrogenase is switched off, the amino acid sequences of subunits B and C being at least 90% identical to the amino acid sequences present in
a) SEQ ID NO:3 (subunit C), and
b) SEQ ID NO:7 (subunit B),
and the amino acid sequence of polypeptide subunit A being identical to the amino acid sequence present in SEQ ID NO:5.

7. Coryneform bacteria according to Claim 6, which carry a deletion, insertion or a base substitution in at least one of subunits A, B and C.

8. Process for the production of L-amino acids, **characterized in that** bacteria according to Claim 6 or 7 are fermented in a suitable medium.

9. Process for the production of L-amino acids according to Claim 8, which comprises concentrating the L-amino acid in the medium or in the cells and isolating the L-amino acid.

10. Process according to Claim 8, **characterized in that** bacteria are used in which additionally further genes of the biosynthetic pathway of the desired L-amino acid are enhanced.

11. Process according to Claim 8, **characterized in that** bacteria are used in which the metabolic pathways which reduce formation of the desired L-amino acid have, at least partially, been eliminated.

12. Process according to one or more of Claims 8 to 11, **characterized in that** L-lysine or L-glutamic acid is produced.

13. Process according to Claims 8-12, **characterized in that** bacteria are fermented in which at the same time one or more of the genes selected from the group consisting of
a) the dapA gene coding for dihydrodipicolinate synthase,
b) the gap gene coding for glyceraldehyde-3-phosphate dehydrogenase,
c) the pyc gene coding for pyruvate carboxylase,
d) the mqo gene coding for malate:quinone oxidoreductase,
e) the lysE gene coding for a lysine export protein,
are enhanced, in particularly overexpressed or amplified.

14. Process according to Claims 8 to 13, **characterized in that** bacteria are fermented in which at the same time one or more of the genes selected from the group consisting of
a) the pck gene coding for phosphoenolpyruvate carboxykinase,
b) the pgi gene coding for glucose-6-phosphate isomerase,
are attenuated.

15. Process according to one or more of the preceding Claims 8 to 14, **characterized in that** microorganisms of the genus Corynebacterium are used.

16. Vector comprising a polynucleotide according to Claim 1 a or 1 d.

17. Vector comprising a polynucleotide according to Claim 4.

18. Coryneform bacteria comprising a vector according to Claim 16 or 17.

## Revendications

1. Polynucléotide isolé de bactéries coryneformes, contenant une séquence polynucléotidique, choisi dans le groupe suivant :
a) un polynucléotide codant pour un polypeptide, qui contient la séquence d'acides aminés de la SEQ ID No. 3,
b) un polynucléotide codant pour un polypeptide, qui contient la séquence d'acides aminés de la SEQ ID No. 5,
c) un polynucléotide codant pour un polypeptide, qui contient la séquence d'acides aminés de la SEQ ID No. 7,
d) un polynucléotide codant pour un polypeptide, qui contient une séquence d'acides aminés qui est identique au moins à 90% à la séquence d'acides aminés de la SEQ ID No. 3,
e) un polynucléotide codant pour un polypeptide, qui contient une séquence d'acides aminés qui est identique au moins à 90% à la séquence d'acides aminés de la SEQ ID No. 7,
les polypeptides selon d) et e) conjointement avec le polypeptide selon b) possédant dans leur action globale l'activité d'une succinate déshydrogénase, et
f) un polynucléotide, qui est complémentaire des polynucléotides en a), b), c), d) ou e).

2. Polynucléotide selon la revendication 1, le polynucléotide étant un ADN recombinant dans les bactéries coryneformes.

3. Polynucléotide selon la revendication 1, le polynucléotide étant un ARN.

4. Polynucléotides selon la revendication 1, choisis dans le groupe suivant :
a) un polynucléotide contenant une séquence nucléotidique choisie parmi la SEQ ID No. 1, la SEQ ID No. 2, la SEQ ID No. 4 ou la SEQ ID No. 6,
b) un polynucléotide contenant une séquence nucléotidique qui correspond à la SEQ ID No. 1, à la SEQ ID No. 2, à la SEQ ID No. 4 ou à la SEQ ID No. 6, dans le cadre de la dégénérescence du code génétique, et
c) un polynucléotide contenant une séquence nucléotidique avec des mutants sens, neutres au plan fonctionnel, de la SEQ ID No. 1, de la SEQ ID No. 2, de la SEQ ID No. 4 ou de la SEQ ID No. 6.

5. Sous-unités polypeptidiques A, B et C qui possèdent dans leur action globale l'activité d'une succinate déshydrogénase, les séquences d'acides aminés des sous-unités polypeptidiques B et C étant identiques au moins à 90% aux séquences d'acides aminés contenues dans :
a) la SEQ ID No. 3 (sous-unité C)
b) la SEQ ID No. 7 (sous-unité B),
et la séquence d'acides aminés de la sous-unité polypeptidique A étant identique à la séquence d'acides aminés contenue dans la SEQ ID No. 5.

6. Bactérie coryneforme isolée, dans laquelle on éteint l'expression d'un ou plusieurs des polynucléotides codant pour les sous-unités polypeptidiques A, B et C qui possèdent dans leur action globale l'activité d'une succinate déshydrogénase, les séquences d'acides aminés des sous-unités B et C étant identiques au moins à 90% aux séquences d'acides aminés contenues dans :
a) la SEQ ID No. 3 (sous-unité C) et
b) la SEQ ID No. 7 (sous-unité B)
et la séquence d'acides aminés de la sous-unité polypeptidique A étant identique à la séquence d'acides aminés contenue dans la SEQ ID No. 5.

7. Bactéries coryneformes selon la revendication 6, qui comprennent une délétion, une insertion ou un échange de bases dans au moins une des sous-unités A, B ou C.

8. Procédé de préparation d'acides aminés L, **caractérisé en ce que** l'on fait fermenter des bactéries selon la revendication 6 ou 7 dans un milieu approprié.

9. Procédé de préparation d'acides aminés L selon la revendication 8, dans lequel on enrichit l'acide aminé L dans le milieu ou dans les cellules et on isole l'acide aminé L.

10. Procédé selon la revendication 8, **caractérisé en ce que** l'on utilise des bactéries dans lesquelles on renforce en plus d'autres gènes de la voie de biosynthèse de l'acide aminé L souhaité.

11. Procédé selon la revendication 8, **caractérisé en ce que** l'on utilise des bactéries dans lesquelles les voies métaboliques qui réduisent la formation de l'acide aminé L souhaité sont au moins partiellement éteintes.

12. Procédé selon une ou plusieurs des revendications 8 à 11, **caractérisé en ce que** l'on prépare de la L-lysine ou de l'acide L-glutamique.

13. Procédé selon les revendications 8 à 12, **caractérisé en ce que** l'on fait fermenter des bactéries dans lesquelles on renforce, en particulier dans lesquelles on surexprime ou on amplifie, simultanément, un ou plusieurs des gènes choisis dans le groupe suivant :
a) le gène dapA codant pour la dihydrodipicolinate synthase,
b) le gène gap codant pour la glycéraldéhyde-3-phosphate déshydrogénase,
c) le gène pyc codant pour la pyruvate carboxylase,
d) le gène mqo codant pour la malate:quinone oxydoréductase et
e) le gène lysE codant pour une protéine d'export de la lysine.

14. Procédé selon les revendications 8 à 13, **caractérisé en ce que** l'on fait fermenter des bactéries dans lesquelles on atténue, simultanément, un ou plusieurs des gènes choisis dans le groupe suivant :
a) le gène pck codant pour la phosphoénolpyruvate carboxykinase et
b) le gène pgi codant pour la glucose-6-phosphate isomérase.

15. Procédé selon une ou plusieurs des revendications 8 à 14 précédentes, **caractérisé en ce que** l'on utilise des microorganismes de la souche Corynebacterium.

16. Vecteur contenant un polynucléotide selon la revendication 1a ou 1d.

17. Vecteur contenant un polynucléotide selon la revendication 4.

18. Bactéries coryneformes contenant un vecteur selon la revendication 16 ou 17.
